# EUROPÄISCHE PATENTSCHRIFT

(11) **EP 3 678 735 B1**
(45) Veröffentlichungstag und Bekanntmachung des Hinweises auf die Patenterteilung: **21.06.2023**
(21) Anmeldenummer: 19805653.3
(22) Anmeldetag: 18.11.2019
(51) Int. Cl.: A61N 1/375, H02J 50/05

(54) **IMPLANTIERBARE ELEKTRISCHE VERBINDERANORDNUNG UND IMPLANTIERBARE ELEKTRODENANORDNUNG**
IMPLANTABLE ELECTRICAL CONNECTOR ARRANGEMENT AND IMPLANTABLE ELECTRODE ARRANGEMENT
ENSEMBLE CONNECTEUR ÉLECTRIQUE IMPLANTABLE ET ENSEMBLE ÉLECTRODE IMPLANTABLE

(30) Priorität: 20.11.2018 DE 102018219831
(43) Veröffentlichungstag der Anmeldung: 15.07.2020
(73) Patentinhaber: Albert-Ludwigs-Universität Freiburg, 79098 Freiburg (DE)
(72) Erfinder: KIELE, Patrick, 79106 Freiburg (DE); PASLUOSTA, Cristian, 79279 Vörstetten (DE); STIEGLITZ, Thomas, 79110 Freiburg (DE)
(74) Vertreter: Grünecker Patent- und Rechtsanwälte PartG mbB
(86) Internationale Anmeldenummer: PCT/EP2019/081630
(87) Internationale Veröffentlichungsnummer: WO 2020/104365

(56) Entgegenhaltungen:
- EP-A2- 2 110 156
- DE-A1-102014 009 136
- DE-A1-102015 108 467
- US-A1- 2015 118 898
- US-A1- 2017 365 948
- US-B2- 9 493 261

## Beschreibung

Die vorliegende Erfindung betrifft eine elektrische Verbinderanordnung, insbesondere für den Einsatz bei implantierbaren Komponenten, und eine zugehörige implantierbare Elektrodenanordnung.

Implantierbare Konnektoren spielen eine wichtige Rolle bei der Herstellung und Implantation von aktiven implantierbaren medizinischen Geräten (Active Implantable Medical Device, AIMD). Diese bestehen üblicherweise aus einem Gehäuse, das eine Steuerelektronik und eine Batterie beinhaltet, implantierbaren Elektroden (oder Elektrodenarrays) und Kabeln für die elektrische Kontaktierung der Elektroden und der Elektronik.

Reversibel lösbare Verbinder (nachfolgend auch als Konnektoren bezeichnet), die in den Kabeln integriert sind, ermöglichen es, die Einzelteile zu separieren. Somit wird sowohl die Implantation erleichtert als auch die Möglichkeit geschaffen, defekte, verbesserte oder verbrauchte Teile (z. B. die Batterie in einem Herzschrittmacher, die nur eine Lebenszeit von 3 bis 7 Jahren hat) auszutauschen.

Derartige Implantate und Verbinder sind z. B. in den Veröffentlichungen J. E. Letechipia, P. H. Peckham, M. Gazdik, and B. Smith, "In-line lead connector for use with implanted neuroprosthesis," IEEE Trans. Biomed. Eng., vol. 38, no. 7, pp. 707-709, 1991; M. Cocco, P. Dario, M. Toro, P. Pastacaldi, and R. Sacchetti, "An implantable neural connector incorporating microfabricated components," J. Micromech. Microeng., vol. 3, no. 4, pp. 219-221, 1993; sowie R. G. Hauser and B. J. Maron, "Lessons from the failure and recall of an implantable cardioverter-defibrillator," (eng), Circulation, vol. 112, no. 13, pp. 2040-2042, 2005; gezeigt.

Je nach gewünschter Anzahl an Kanälen sowie der gewünschten Integrationsdichte nimmt die Größe des Konnektors zu, was wiederum negative Auswirkungen auf das umliegende Gewebe haben kann. Bei klassischen Konnektor-Konzepten ist die Einbringkraft (Englisch: insertion force) proportional zur Anzahl der gewünschten Kontakte. Bei miniaturisierten implantierbaren Konnektoren stellt außerdem die elektrische Isolation zwischen den einzelnen benachbarten Kontakten unterschiedlicher Kanäle ein Problem dar, da durch den Einsatz im Körper eine gesättigte 100%ig feuchte Umgebung vorherrscht. Die elektrische Isolierung muss zusätzlich realisiert werden und benötigt zusätzliche Materialien und Kräfte. Darüber hinaus muss, um eine ausrechende elektrische Isolation sicherzustellen, bei bekannten Verbinderanordnungen die Distanz zwischen benachbarten Kontakten vergleichsweise groß sein, so dass die Integrationsdichte nicht ausreichend hoch ist. Die Anzahl an Kanälen ist daher relativ begrenzt und liegt bei bekannten Anordnungen bei maximal 16.

Nach dem Zusammenfügen beider Konnektor-Partner muss bei einigen bekannten Verbinderanordnungen ein Klebstoff aufgebracht werden um eine Isolation nach außen herzustellen. Ein Beispiel ist der "Craggs connector" von Finetech Medical, siehe P. E. Donaldson, "The Craggs connector: A termination for Cooper cable," (eng), Medical & biological engineering & computing, vol. 23, no. 2, pp. 195-196, 1985.

Die DE 10 2015 108467 A1 offenbart ein Gehäuse für ein medizinisches Implantat. Das Gehäuse umfasst eine Gehäusewand aus einem elektrisch isolierenden Material mit einer Innenseite sowie einer Außenseite und eine Stromdurchleitung durch die Gehäusewand. Die Stromdurchleitung umfasst eine auf der Innenseite der Gehäusewand angeordnete elektrisch leitende erste Anschlusskontaktfläche, eine auf der Außenseite der Gehäusewand angeordnete elektrisch leitende zweite Anschlusskontaktfläche, einen in die Gehäusewand integrierten Kondensator mit einer ersten Kondensatorelektrode und einer zweiten Kondensatorelektrode, eine erste Verbindungsleitung, welche die erste Anschlusskontaktfläche mit der ersten Kondensatorelektrode elektrisch verbindet, und eine zweite Verbindungsleitung, welche die zweite Anschlusskontaktfläche mit der zweiten Kondensatorelektrode elektrisch verbindet. Zwischen der ersten Anschlusskontaktfläche und der zweiten Anschlusskontaktfläche besteht keine durchgehend elektrisch leitende Verbindung.

Die vorliegende Erfindung stellt sich die Aufgabe, eine implantierbare Verbinderanordnung anzugeben, die es erlaubt, auch dicht gepackte Kontaktarrays zuverlässig und elektrisch wie mechanisch langzeitstabil zu verbinden. Dabei soll die Verbinderanordnung möglichst kostengünstig und darüber hinaus biokompatibel und zertifizierbar für den chronischen Einsatz sein.

Diese Aufgaben werden durch den Gegenstand des unabhängigen Patentanspruchs gelöst.

Vorteilhafte Weiterbildungen der vorliegenden Erfindung sind Gegenstand mehrerer abhängiger Patentansprüche.

Die vorliegende Erfindung basiert auf der Idee, dass eine kapazitive Kopplung (Verschiebungsstrom) an Stelle einer galvanisch-faradischen Übertragung (Leitungsstrom) eingesetzt wird. Darüber hinaus haben die Erfinder der vorliegenden Erfindung erkannt, dass in vorteilhafter Weise bei einer kapazitiven Kopplung ein definierter Trennspalt vorgesehen werden kann, der sich im Betrieb mit Wasser oder Elektrolyt füllt. Eine solche Wasserschicht mit einer hohen relativen Dielektrizitätszahl stellt ein gutes Dielektrikum für die kapazitive Kopplung dar. Darüber hinaus müssen keinerlei Maßnahmen ergriffen werden, um ein Eindringen von Flüssigkeit in die Verbinderanordnung zu verhindern. Die Langzeitstabilität der Verbindung wird damit erhöht und die Wahrscheinlichkeit für Ausfälle reduziert.

Insbesondere weist eine implantierbare elektrische Verbinderanordnung zum elektrischen Verbinden mindestens einer ersten und einer zweiten elektrischen Komponente einen ersten Verbinder mit mindestens einem ersten Anschluss und mindestens einer ersten Kopplungselektrode, die mit dem ersten Anschluss verbunden ist, und einen zweiten Verbinder mit mindestens einem zweiten Anschluss und mindestens einer zweiten Kopplungselektrode, die mit dem zweiten Anschluss verbunden ist, auf.

Der erste Verbinder und der zweite Verbinder sind so miteinander verbindbar, dass der erste und der zweite Anschluss über die erste Kopplungselektrode und die zweite Kopplungselektrode kapazitiv verbindbar sind, wobei im verbundenen Zustand zwischen der ersten Kopplungselektrode und der zweiten Kopplungselektrode ein definierter Trennspalt gebildet ist.

Durch diese Art der Verbindung muss keine Anpresskraft aufgebracht werden, um eine elektrische Verbindung herzustellen. Vielmehr ist ein Spalt mit genau festgelegten Abmessungen zwischen den beiden Kontakten vorgesehen. Gemäß einer vorteilhaften Weiterbildung der vorliegenden Erfindung ist der Trennspalt nicht hermetisch gegenüber der Außenumgebung abgeschlossen. Wird die Verbinderanordnung implantiert, kondensiert Wasser in dem Trennpalt. Durch die deutlich höhere Dieelektrizitätszahl von Wasser (ε_{r,H2O} = 80) im Vergleich zu Luft (ε_{r,Luft} = 1) wird die kapazitive Kopplung über den Spalt unterstützt.

Im Gegensatz zu ohmschen Kontakten müssen die elektrisch leitfähigen Kopplungselektroden nicht geöffnet werden, d. h. eine isolierende Materialschicht kann darauf verbleiben. Diese kann z. B. aus Polymeren (Parylene-C, PDMS), Oxiden (TiOx) oder anderen Materialien bestehen. Parylene-C ist beispielsweise ein für Humanimplantationen zugelassenes Material und ist bei einer Dicke von 10 µm elektrisch dicht. Diverse Oxide erreichen diese elektrische Dichtigkeit bereits bei deutlich niedrigeren Schichtdicken und weisen eine höhere Dielektrizitätszahl auf (z.B. ε_{rTiO2} = 63,7; ε_{r,parylene-C} = 3,1). Beide Faktoren versprechen eine Steigerung der Kopplungskapazität und dementsprechend eine bessere Kopplung. Wird keine Isolierung verwendet, kann die elektrische Kopplung kapazitiv und resistiv über eingelagertes Wasser erfolgen. In diesem Fall müssen allerdings benachbarte Kontakte gegeneinander isoliert werden.

Ein Übersprechen zwischen benachbarten Kanälen entsteht bei ohmschen Kontakten üblicherweise aufgrund von elektrischen Leckpfaden durch Einlagerung von Wasser in unbeabsichtigt vorhandenen Leerräumen. Dadurch, dass bei dem erfindungsgemäßen Konzept eine kapazitive Kopplung verwendet wird, wird ein solcher resisitver Leckpfad zu benachbarten Kanälen unterbunden.

Gemäß einer vorteilhaften Weiterbildung der vorliegenden Erfindung sind die Kopplungselektroden lokal mit einem elektrisch isolierenden Material beschichtet, insbesondere mit einem Material, das sich von dem übrigen Kapselungsmaterial des Verbinders unterscheiden kann aber nicht muss (je nach Bauform genügt es, unterschiedliche Dicken für das isolierende Material und das Kapselungsmaterial vorzusehen, um eine gerichtete kapazitive Kopplung zu erreichen). Auf diese Weise kann das Dielektrikum zwischen den beiden Kopplungselektroden für eine besonders effiziente kapazitive Kopplung optimiert werden.

Generell wählt man als Isolatormaterial für die Anwendung in implantierbaren Komponenten üblicherweise Silikonkautschuk (Polydimethylsiloxan, PDMS). PDMS hat die ausreichende Langzeitstabilität, die für den jahrelangen Gebrauch in wässriger oder feuchter Umgebung, wie sie für eine aktive implantierbare Baugruppe auftritt, gefordert werden muss. Für den Fall, dass der Trennspalt wenigstens teilweise von einem Silikonmaterial begrenzt ist, konnte gezeigt werden, dass durch Osmoseeffekte im implantierten Zustand Wasser aus dem Silikonmaterial austritt und den Trennspalt füllt. Dadurch kann in vorteilhafter Weise ein definiertes Dielektrikum zwischen den miteinander verbundenen Kopplungselektroden erreicht werden.

Gemäß einer vorteilhaften Weiterbildung der vorliegenden Erfindung weist die elektrische Verbindunganordnung weiterhin eine erste Justierstruktur auf, die an dem ersten Verbinder angeordnet ist, und eine zweite Justierstruktur, die an dem zweiten Verbinder angeordnet ist, wobei die erste und die zweite Justierstruktur zum Einstellen einer Breite des Trennspalts im verbundenen Zustand zusammenwirken. Solche Justierstrukturen, die man auch als A-lignmentstrukturen bezeichnen kann, können beispielsweise Vorsprünge an einem Verbinder angeordnet sein, die mit Ausnehmungen an dem anderen Verbinder zusammenwirken. Die Justierstrukturen erleichtern zum einen das Zusammenführen der beiden Verbinder und vermeiden zum anderen ein Verrutschen der geschlossenen Verbindung im Betrieb.

Da die elektrische Kopplung der beiden Verbinder kapazitiv erfolgt, werden niedrige Frequenzen geblockt. Um bei der Anwendung mit einer Stimulationselektrode trotzdem niedrige Stimulationsfrequenzen an der Elektrode zu erhalten, kann ein zusätzlicher Glättungskondensator integriert werden.

Gemäß einer vorteilhaften Weiterbildung der vorliegenden Erfindung weist der erste Verbinder und/oder der zweite Verbinder ein elektrisch isolierendes Substrat auf. Das Substratmaterial beider Verbinder kann je nach Ausführung flexibel oder starr sein, sollte aber nicht elektrisch leitend sein. Bei elektrisch leitendem Material müssen zusätzliche Isolationsschichten eingebracht werden. Es eignen sich beispielsweise Polyimid, PDMS, Keramik sowie alle anderen Materialien, die für elektrische Schaltungsträger eingesetzt werden.

Die Vorteile der vorliegenden Erfindung kommen besonders dann zum Tragen, wenn der erste Verbinder ein Array von ersten Kopplungselektroden aufweist, die im verbundenen Zustand mit einem Array von zweiten Kopplungselektroden verbunden sind. Wie bereits oben erwähnt, können hohe Integrationsdichten erreicht werden, da benachbarte Elektroden nicht durch resistive Leckstrompfade verbunden sind.

Die vorliegende Erfindung ist nicht auf eine Verbinderanordnung beschränkt, die nur zwei Verbinder aufweist. Vielmehr kann auch ein mehrlagiger Aufbau gewählt werden. Beispielsweise kann die elektrische Verbinderanordnung weiterhin einen dritten Verbinder, mit mindestens einem dritten Anschluss und einer dritten Kopplungselektrode, die mit dem dritten Anschluss verbunden ist, aufweisen, wobei der zweite Verbinder mindestens einen vierten Anschluss und eine vierte Kopplungselektrode, die mit dem vierten Anschluss verbunden ist, aufweist, und wobei die dritte und vierte Kopplungselektrode im verbundenen Zustand der Verbinderanordnung kapazitiv gekoppelt sind und zwischen der dritten und vierten Kopplungselektrode ein zweiter Trennspalt ausgebildet ist. Auf diese Weise kann die Integrationsdichte und die Anzahl der übertragbaren Kanäle weiter erhöht werden.

Insbesondere kann vorgesehen sein, dass jeder der Verbinder ein Substrat mit darauf angeordneten Kopplungselektroden aufweist, und wobei der zweite Verbinder auf einer ersten Seite des Substrats die zweiten Kopplungselektroden trägt und auf einer der ersten Seite gegenüberliegenden zweiten Seite die vierten Kopplungselektroden trägt.

Gemäß einer weiteren vorteilhaften Ausgestaltung der vorliegenden Erfindung kann vorgesehen sein, dass der erste Verbinder und der zweite Verbinder eingerollt sind, um jeweils eine zylindrische Raumform zu haben. Der erste und der zweite Verbinder werden zum Schließen der Verbindung ineinander geschoben, bis die Kopplungselektroden zum ausbilden eines vollständigen Kondensators einander gegenüberstehen. Diese Anordnung ist besonders platzsparender und lässt sich speziell mit einer kapazitiven Verbinderanordnung gut realisieren, da beide Verbinder eine völlig glatte Oberfläche aufweisen.

Alternativ kann auch vorgesehen sein, dass der erste Verbinder mindestens einen stiftförmigen Vorsprung aufweist, an dem die mindestens eine erste Kopplungselektrode angeordnet ist, und wobei der zweite Verbinder mindestens eine Ausnehmung zur wenigstens teilweisen Aufnahme des stiftförmigen Vorsprungs aufweist, wobei in der Ausnahme die mindestens eine zweite Kopplungselektrode angeordnet ist.

In vorteilhafter Weise können weitere elektronische Komponenten in einem oder beiden der Verbinder integriert sein. Dies können elektronische Ansteuerungs- und Auswertungsschaltungen, aber auch passive Komponenten wie weitere Widerstände, Kondensatoren und Spulen sein.

Die vorliegende Erfindung bezieht sich weiterhin auf eine implantierbare Elektrodenanordnung, die mindestens eine Verbinderanordnung gemäß der vorliegenden Erfindung verwendet. Insbesondere für stimulierende Elektroden, die einen elektrischen Treiber vorsehen, ist das erfindungsgemäße Konzept besonders vorteilhaft. Die implantierbare Elektrodenanordnung gemäß der vorliegenden Erfindung umfasst mindestens eine implantierbare Elektrode und eine implantierbare Verbinderanordnung gemäß der vorliegenden Erfindung, wobei der mindestens eine erste Anschluss jeweils mit der mindestens einen Elektrode verbunden ist und der mindestens eine zweite Anschluss mit einer Ansteuerschaltung verbindbar ist.

Wie bereits erwähnt, überträgt die kapazitive Kopplung nur höherfrequente Wechselsignale, so dass in vorteilhafter Weise zwischen der mindestens einen implantierbaren Elektrode und der ersten Kopplungselektrode ein Glättungskondensator angeordnet werden kann. Die Ansteuerschaltung gibt ein Anregungssignal aus, das eine höhere Frequenz hat als ein Signal, das an die implantierbare Elektrode ausgegeben wird.

Zum besseren Verständnis der vorliegenden Erfindung wird diese anhand der in den nachfolgenden Figuren dargestellten Ausführungsbeispiele näher erläutert. Dabei werden gleiche Teile mit gleichen Bezugszeichen und gleichen Bauteilbezeichnungen versehen. Weiterhin können auch einige Merkmale oder Merkmalskombinationen aus den gezeigten und beschriebenen unterschiedlichen Ausführungsformen für sich eigenständige, erfinderische oder erfindungsgemäße Lösungen darstellen. Dabei zeigen:
- **FIG. 1**: eine schematische Schnittdarstellung einer kapazitiven Verbinderanordnung vor dem Schließen der Verbindung;
- **FIG. 2**: eine schematische Schnittdarstellung der kapazitiven Verbinderanordnung aus Fig. 1 nach dem Schließen der Verbindung;
- **FIG. 3**: eine schematische Schnittdarstellung einer alternativen kapazitiven Verbinderanordnung vor dem Schließen der Verbindung;
- **FIG. 4**: eine schematische Schnittdarstellung der kapazitiven Verbinderanordnung aus Fig. 3 nach dem Schließen der Verbindung;
- **FIG. 5**: eine schematische Schnittdarstellung einer alternativen kapazitiven Verbinderanordnung vor dem Schließen der Verbindung;
- **FIG. 6**: eine schematische Schnittdarstellung der kapazitiven Verbinderanordnung aus Fig. 5 nach dem Schließen der Verbindung;
- **FIG. 7**: eine schematische perspektivische Darstellung einer alternativen kapazitiven Verbinderanordnung vor dem Schließen der Verbindung;
- **FIG. 8**: eine schematische Schnittdarstellung einer weiteren kapazitiven Verbinderanordnung nach dem Schließen der Verbindung;
- **FIG. 9**: eine schematische Schnittdarstellung einer mehrlagigen kapazitiven Verbinderanordnung nach dem Schließen der Verbindung;
- **FIG. 10**: eine schematische perspektivische Darstellung einer weiteren kapazitiven Verbinderanordnung;
- **FIG. 11**: eine schematische perspektivische Darstellung einer weiteren kapazitiven Verbinderanordnung;
- **FIG. 12**: eine schematische perspektivische Darstellung einer weiteren kapazitiven Verbinderanordnung;
- **FIG. 13**: eine schematische perspektivische Darstellung einer weiteren kapazitiven Verbinderanordnung;
- **FIG. 14**: eine schematische Schnittdarstellung einer weiteren kapazitiven Verbinderanordnung.

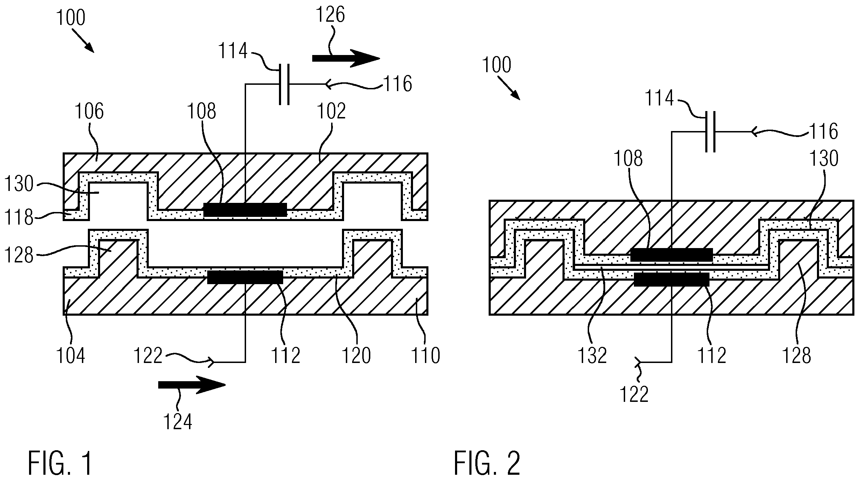

Mit Bezug auf die Figuren 1 und 2 soll nachfolgend zunächst das Prinzip der Verbinderanordnung 100 gemäß einer ersten vorteilhaften Ausführungsform der vorliegenden Erfindung näher erläutert werden. Figur 1 zeigt eine perspektivische Ansicht einer Verbinderanordnung 100 im noch nicht verbundenen Zustand. Die Verbinderanordnung 100 weist einen ersten Verbinder 102 und einen zweiten Verbinder 104 auf. Der erste Verbinder 102 hat ein erstes Substrat 106, an dem mindestens eine erste Kopplungselektrode 108 angeordnet ist. Der zweite Verbinder 102 umfasst ein zweites Substrat 110 mit mindestens einer zweiten Kopplungselektrode 112. Die erste Kopplungselektrode 108 ist über einen Glättungskondensator 114 mit einem ersten Anschluss 116 verbunden. Selbstverständlich ist der Glättungskondensator 114 nur optional. Der erste Anschluss 116 kann beispielsweise mit einer Stimulationselektrode verbunden werden (in den Figuren nicht gezeigt).

Die zweite Kopplungselektrode 112 ist elektrisch mit einem zweiten Anschluss 122 verbunden. Der zweite Anschluss 122 kann beispielsweise mit einer Treiberelektronik (in den Figuren nicht gezeigt) verbunden werden.

Im verbundenen Zustand bilden die erste Kopplungselektrode 108 und die zweite Kopplungselektrode 112 die beiden Platten eines Kondensators aus. Ein Eingangssignal 124 kann an dem ersten Anschluss 122 eingespeist werden, und ein Ausgabesignal 126 kann entsprechend über die beiden Kopplungselektroden 108, 112 und den Glättungskondensator 114 an den Anschluss 116 ausgegeben werden.

Die Kopplungselektroden 108, 112 können durch Legierungen wie Platin-Iridium oder MP35N^{®} gebildet sein. MP35N^{®} ist ein eingetragenes Warenzeichen von Standard Pressed Steel Technologies, Inc. Die Bestandteile von MP35N^{®} sorgen für eine ausgezeichnete Korrosionsbeständigkeit der Legierung, da alle vier Legierungsbestandteile Nickel, Kobalt, Chrom und Molybdän die Korrosionsbeständigkeit in fast allen in der Industrie gebräuchlichen Edelstahl-, Nickel- und Kobaltlegierungen steigern. Der Anteil von 20% Chrom verbessert die Beständigkeit gegen Oxidation, Sulfidierung und chemische Reaktionen mit Salz bei erhöhten Temperaturen. Jedes andere elektrisch leitfähige Elektrodenmaterial kann selbstverständlich ebenfalls verwendet werden.

Zur elektrischen Isolierung und zum Schutz gegen die äußere Umgebung ist eine elektrisch isolierende Passivierungsschicht 118, 120 am ersten bzw. zweiten Verbinder 102, 104 vorgesehen.

Ein Justiervorsprung 128, der beispielsweise kreisförmig oder rechteckförmig den eigentlichen Kontaktbereich um die Kopplungselektrode 112 umgibt, ist vorgesehen, um mit einer zugehörigen Justierausnehmung 130 zum Ausrichten des ersten und zweiten Verbinder 102, 104 mit Bezug aufeinander zusammenzuwirken.

Figur 2 zeigt die erfindungsgemäße kapazitive Verbinden der Anordnung im zusammengesteckten Zustand. Der Justiervorsprung 128 ist in der Justierausnehmung 130 aufgenommen und die erste Kopplungselektrode 108 liegt der zweiten Kopplungselektrode 112 gegenüber, so dass ein Kopplungskondensator ausgebildet ist.

Erfindungsgemäß sind die Abmessungen der Justierausnehmung 130 und des Justiervorsprungs 128 so gewählt, dass im verbundenen Zustand zwischen der ersten Passivierungsschicht 118 und der zweiten Passivierungsschicht 120 im Bereich der Kopplungselektroden 108, 112 ein definierter Trennspalt 132 verbleibt. Dieser Trennspalt kann erfindungsgemäß im Betrieb mit Wasser gefüllt sein, so dass zwischen den beiden Kopplungselektroden 108, 112 ein verbessertes Dielektrikum entsteht. Dabei füllt sich der Trennspalt 132 im Betrieb mit Flüssigkeit aus dem umgebenden Medium und/oder mit Wasser, das aus der ersten und zweiten Passivierungsschicht 118, 120 austritt. Insbesondere, wenn die Passivierungsschicht nun 118, 120 aus einem Silikonmaterial bestehen, bildet sich durch Osmoseeffekte im implantierten Zustand Kondenswasser in dem Trennspalt 132.

Wie schematisch in Figur 2 angedeutet, hat die erste Kopplungselektrode 108 eine geringfügig größere Fläche als die zweite Kopplungselektrode 112 um eventuelle Justierfehler auszugleichen und eine zuverlässige Kopplung sicherzustellen.

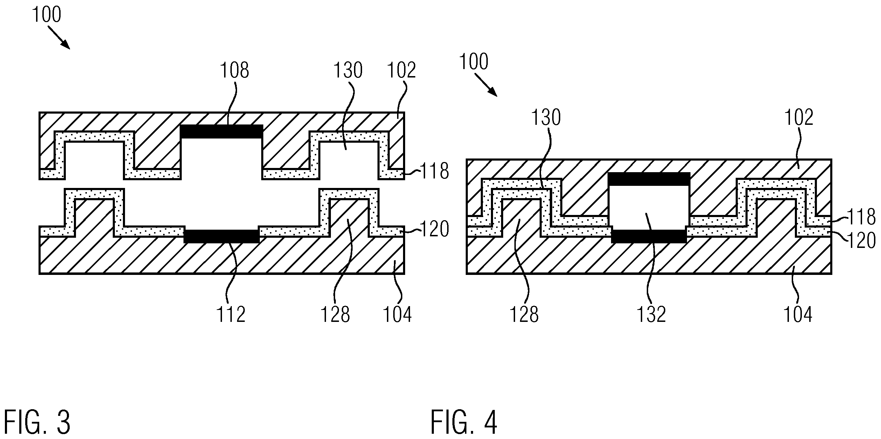

Mit Bezug auf die Figuren 3 und 4 wird nachfolgend eine weitere vorteilhafte Ausführungsform der erfindungsgemäßen Verbinderanordnung 100 erläutert. Dabei sind die Zuleitungen zu den Anschlüssen 116, 122, die sich in der Realität quer zur Zeichenebene befinden, und die Anschlüsse selbst nicht dargestellt. Figur 3 zeigt die Verbinderanordnung im nicht verbundenen Zustand, während Figur 4 die Verbinderanordnung im zusammengesteckten Zustand zeigt.

Gemäß dieser speziellen Ausführungsform sind die erste Kopplungselektrode 108 und die zweite Kopplungselektrode 112 nicht von einer Passivierungsschicht bedeckt. Weiterhin ist wenigstens die erste Kopplungselektrode 108 in das Substrat 106 zurückgesetzt, um einen noch breiteren Trennspalt 132 zu erzeugen. Da keine ohmsche Signalübertragung erfolgt, müssen die Kopplungselektroden 108, 112 nicht zwingend von einer Isolierschicht bedeckt sein, solange ein Übersprechen zu benachbarten Kopplungselektroden verhindert wird.

Auch bei dieser Ausführungsform hat die erste Kopplungselektrode 108 eine geringfügig größere Fläche als die zweite Kopplungselektrode 112 um eventuelle Justierfehler auszugleichen und eine sichere Signalübertragung zu gewährleisten.

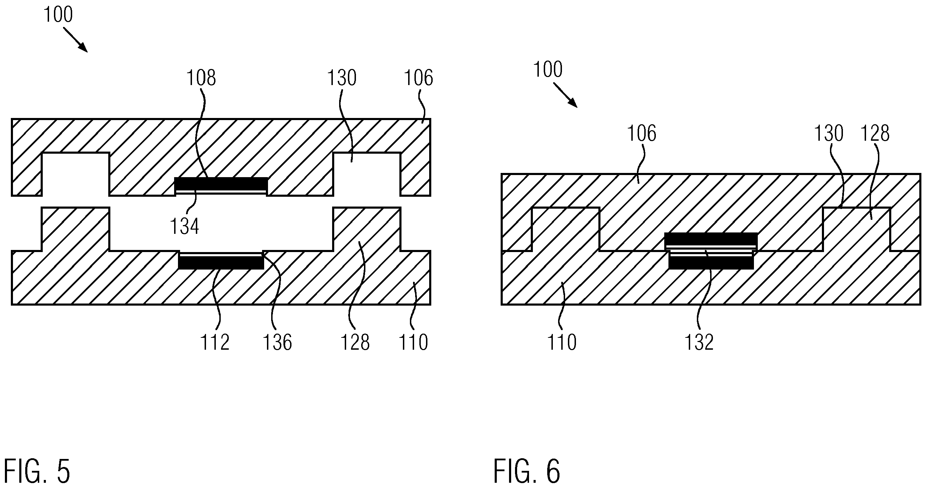

Die Figuren 5 und 6 illustrieren eine weitere vorteilhafte Ausführungsform einer Verbinderanordnung 100 gemäß der vorliegenden Erfindung. Dabei zeigt Figur 5 die Verbinderanordnung 100 im nicht verbundenen Zustand, während Figur 6 die Verbinderanordnung 100 im verbundenen Zustand zeigt

Gemäß dieser alternativen Ausführungsform sind die ersten und zweiten Substrate 106, 110 nicht flächendeckend von einer Passivierungsschicht bedeckt. Stattdessen ist lokal an der ersten Kopplungselektrode eine erste lokale Dielektrikumsschicht 134 und an der zweiten Kopplungselektrode 112 eine zweite lokale Dielektrikumsschicht 136 vorgesehen. Die Dicken der beiden Dielektrikumsschichten 134, 136 sind so gewählt, dass im Zusammenspiel mit den Justiervorsprüngen 128 und der Justierausnehmung 130 ein definierter Spalt 132, der sich im Betrieb mit Flüssigkeit füllt, verbleibt.

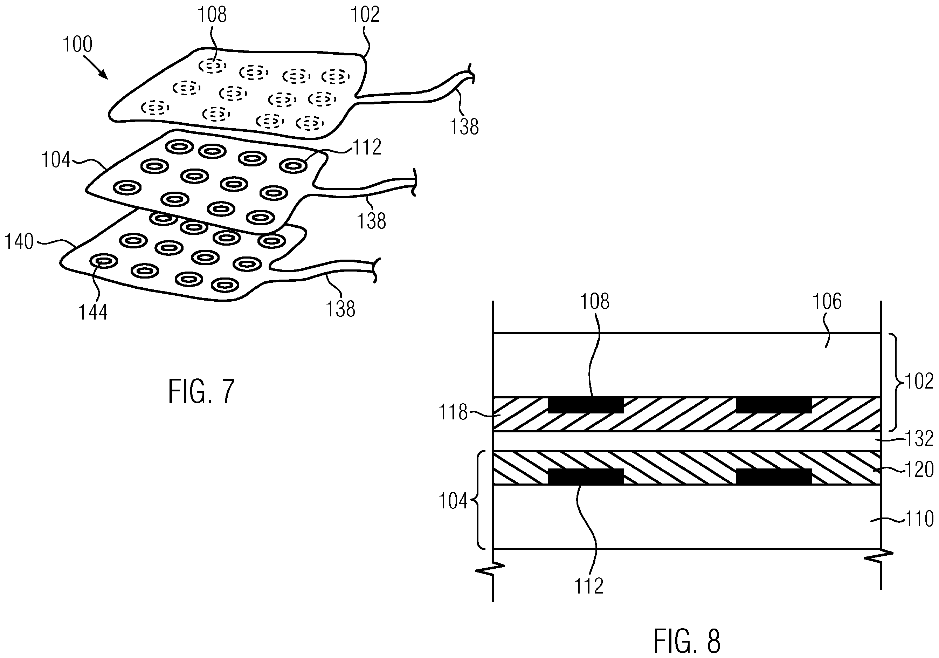

Figur 7 zeigt in einer schematischen perspektivischen Ansicht eine implantierbare Verbinderanordnung 100, die eine Vielzahl von kapazitiven Kontaktanordnungen gemäß der vorliegenden Erfindung umfasst. Insbesondere sind ein erster Verbinder 102 und ein zweiter Verbinder 104 vorgesehen, die jeweils über ein Array von Kopplungselektroden 108, 112 elektrisch miteinander verbindbar sind. Jeder der Verbinder 102, 104 weist eine Leitung 138 auf, die den in den Figuren 1 bis 6 gezeigten Anschlüssen entspricht. Die Substrate der ersten und zweiten Verbinder 102, 104 sind in vorteilhafter Weise flexibel, um sich ihrer Umgebung im implantierten Zustand mechanisch anpassen zu können.

Gemäß einer ersten Ausführungsform umfasst die kapazitive Verbinderanordnung nur den ersten und den zweiten Verbinder 102, 104. In diesem Fall sieht ein schematischer Querschnitt wie in Fig. 8 dargestellt aus.

Der erste Verbinder 102 umfasst dabei ein erstes Substrat 106, dass vorzugsweise aus einem flexiblen elektrisch isolierenden Material hergestellt ist. Auf dem Substrat 106 sind ein Array von ersten Kopplungselektroden 108 angeordnet, die jeweils mit elektrischen Zuleitungen verbunden sind, die zu der elektrischen Leitungen 138 führen (in der Figur nicht sichtbar). Die Elektroden 108 können entsprechend jeder der Ausführungsformen nach den Figuren 1 bis 6 ausgestaltet sein.

Insbesondere ist beispielsweise eine erste Passivierungsschicht 118 vorgesehen, die die Kopplungselektroden 108 flächig bedeckt. Der zweite Verbinder 104 hat ebenfalls ein flexibles elektrisch isolierendes Substrat 110 und zweite Kopplungselektroden 112, die in einem Array angeordnet sind, das dem Array der ersten Kopplungselektroden 108 entspricht. Eine zweite Passivierungsschicht 120 deckt die Kopplungselektroden 112 flächig ab. Durch entsprechende Abstandshalter (in den Figuren 7 und 8 nicht gezeigt) wird sichergestellt, dass ein Trennspalt 132 zwischen dem ersten Verbinder 102 und dem zweiten Verbinder 104 verbleibt.

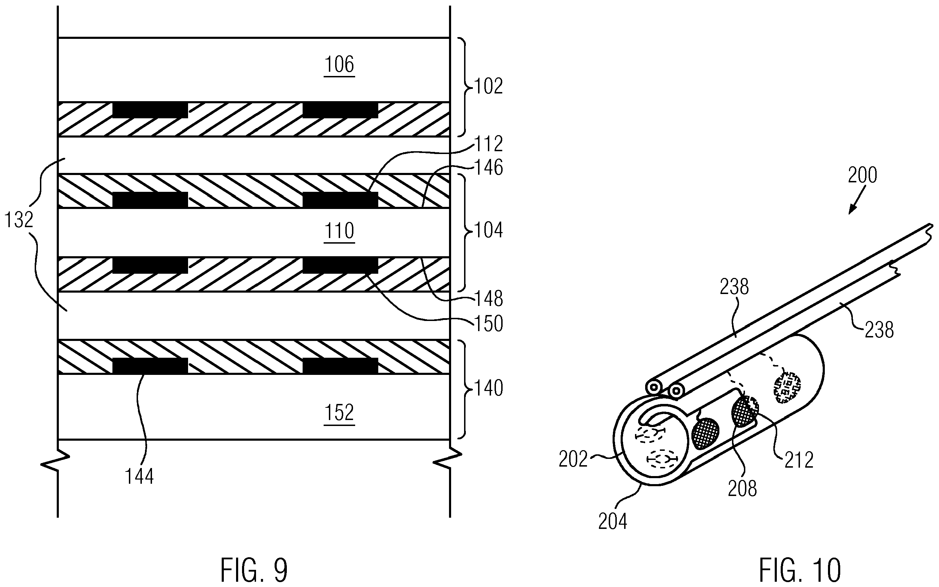

Gemäß einer weiteren vorteilhaften Ausführungsform kann weiterhin vorgesehen sein, dass der zweite Verbinder 104 auch an seiner Rückseite Kopplungselektroden aufweist. Dann kann, wie in Fig. 7 gezeigt, ein dritter Verbinder 140 mit den rückseitigen Kopplungselektroden 142 verbunden werden. Diese Anordnung ist als schematische Schnittdarstellung in Figur 9 gezeigt.

Gemäß dieser Ausführungsform besitzt der zweite Verbinder 104 sowohl auf einer ersten Seite 146 des Substrats 110, wie auch auf einer zweiten Seite 148 des Substrats 110 kapazitive Kopplungselektroden 150. Der dritte Verbinder 140 ist analog zu dem ersten Verbinder 102 aufgebaut und weist ein drittes Substrat 152 mit dritten kapazitiven Kopplungselektroden 144 auf. Ein Trennspalt 132 ist erfindungsgemäß auch zwischen den rückseitigen Kopplungselektroden 150 und den dritten Kopplungselektroden 144 vorgesehen.

Die in Figur 7 und 9 gezeigte Mehrlagenanordnung hat den Vorteil, dass eine noch höhere Integrationsdichte erreicht werden kann. Gleichzeitig reduziert sich die beanspruchte Fläche.

Neben den einfachen planaren Anordnungen, die mit Bezug auf die Figuren 1 bis 9 erläutert wurden, können aber die erfindungsgemäßen Prinzipien auch für verschiedene dreidimensionale Geometrien vorteilhaft angewendet werden.

Beispielsweise zeigt Figur 10 eine kapazitive Verbinderanordnung 200, bei welcher der erste Verbinder 202 und der zweite Verbinder 204 aufgerollt sind, um eine im wesentlichen zylindrische Gestalt anzunehmen. Die Kopplungselektroden 208, 212 werden dabei einander überlagernd angeordnet, sobald die beiden Zylinder ineinander geschoben werden.

Eine Herstellung dieser Anordnung 200 kann beispielsweise dadurch erfolgen, dass die Kopplungselektrodenarrays mit einer mechanischen Vorspannung gefertigt werden, so dass sie sich mit einem definierten Radius einrollen. Die Radien werden so gewählt, dass das äußere Array eine Kraft nach innen aufbringt und das innere Array dieser Kraft entgegenwirkt. Dadurch wird auf alle Kontakte ein definierter Druck aufgebracht.

Selbstverständlich wird auch bei dieser Anordnung über entsprechende Justiermittel oder über eine entsprechende Strukturierung der Kontakte, wie beispielsweise in Figur 6 gezeigt, ein Trennspalt zwischen den Kopplungselektroden eingestellt. Zuleitungen 238 erlauben den Anschluss externer Komponenten.

Gemäß einer weiteren vorteilhaften Ausführungsform der implantierbaren Verbinderanordnung 300 können die ersten Kopplungselektroden 308 auch eine ringförmige Gestalt haben und auf stiftförmigen Vorsprüngen 354 angeordnet sein. Entsprechend sind dann ebenfalls ringförmige zweite Kopplungselektroden 312 im Inneren von buchsenförmigen Ausnehmungen 356 angeordnet. Durch Zusammenstecken der beiden Verbinder 302, 304 werden die Kopplungselektroden 308, 312 kapazitiv miteinander verbunden. Zuleitungen 338 erlauben die Verbindung zu den jeweiligen zu kontaktierenden Komponenten (in der Figur nicht gezeigt). Durch die spezielle geometrische Ausgestaltung der stiftförmigen Vorsprünge 354 und der Ausnehmungen 356 wird in radialer Richtung ein definierter Spalt zwischen den ringförmigen Kopplungselektroden 308 und 312 eingestellt.

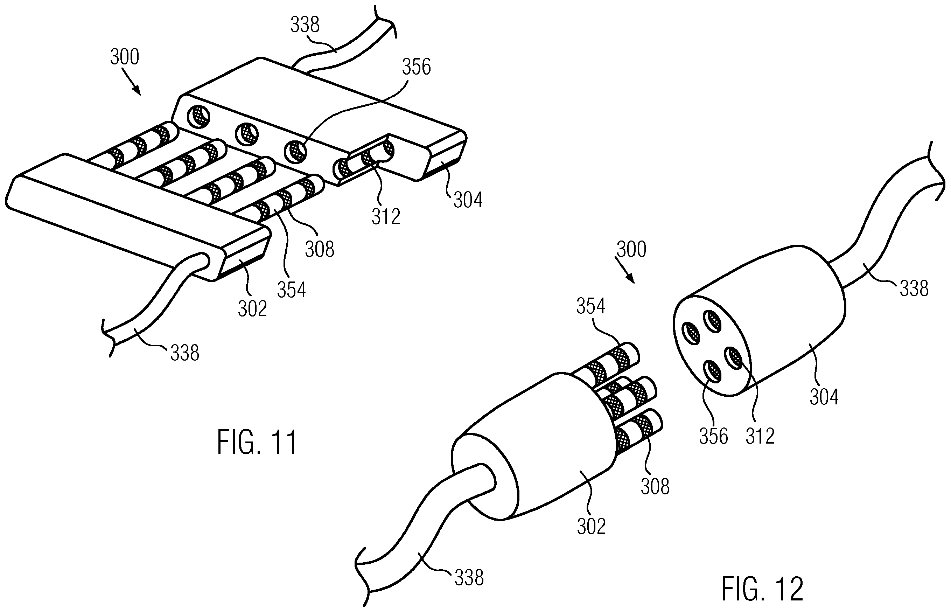

Selbstverständlich können die räumlichen Geometrien der Verbinder 302, 304 auch völlig anders gestaltet sein. Ein weiteres Beispiel für einen solchen dreidimensionalen steckverbinderähnlichen Aufbau ist in Figur 12 illustriert. Der Vorteil einer solchen Anordnung kann darin gesehen werden, dass die Verbindung mechanisch vergleichsweise stabil ist. Durch das Vorsehen des Trennspalts und die kapazitive Kopplung sind keine besonders hohen Steckkräfte erforderlich und eine Abdichtung gegenüber der feuchten Umgebung ist im implantierten Zustand nicht nötig.

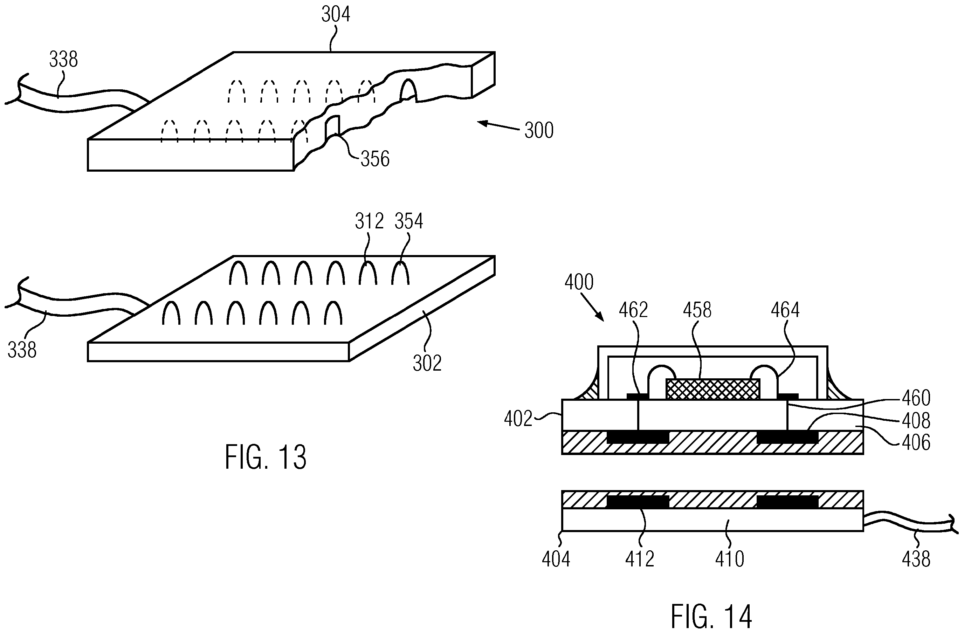

Eine weitere Variante der Verbinderanordnung 300 mit Stiften und Buchsen ist schließlich in Figur 13 gezeigt. Bei dieser Ausgestaltung ist jeder Vorsprung mit nur einer kapazitiven Kopplungselektrode versehen.

Figur 14 zeigt eine weitere vorteilhafte Ausgestaltung einer Verbinderanordnung 400. Wie bereits erwähnt, können in einem oder auch beiden der Verbinder 402, 404 weitere elektrische oder elektronische Bauelemente integriert sein. Figur 14 zeigt beispielhaft die Integration einer elektronischen Komponente 458 in einer Verbinderanordnung 400. In der gezeigten Verbinderanordnung 400 ist der erste Verbinder 402 auf der den Kopplungselektroden 408 abgewandten Seite des Substrats 406 mit einer elektronischen Komponente 458 versehen. Die Kopplungselektroden 408 sind über metallisierte Vias 460 mit Bondpads 462 verbunden. Die elektronische Komponente 458 kann über Bonddrähte 464 mit den Bondpads 462 verbunden werden. Ein Gehäuse 466 oder eine Vergussmasse kann die elektronische Komponente 458 verkapseln.

Selbstverständlich können aber auch Surface Mount Device (SMD)-Komponenten (oder jede andere geeignete Aufbau- und Verbindungstechnik) zum Einsatz kommen.

Der zweite Verbinder 404 ist durch ein entsprechendes planares Array von kapazitiven Kopplungselektroden 412 auf einem Substrat 410 gebildet. Eine elektrische Leitung 438 erlaubt den Anschluss externer Komponenten (in den Figuren nicht gezeigt).

Zusammenfassend stellt die vorliegende Erfindung eine kapazitive, implantierbare, reversible und mehrkanalige Verbindung bereit. Dabei wird kondensiertes bzw. eindringendes Wasser an der Kopplungsstelle als gewünschte Effekt zur kapazitive Kopplung zweier Verbinder genutzt. In vorteilhafter Weise bietet diese Anordnung die Skalierbarkeit der Anzahl der elektrischen Kanäle bei gleichbleibender Einbringkraft. Somit kann eine mehrkanalige, reversible und implantierbare Verbindung ohne die Notwendigkeit von elektrischen Isolierungsstrukturen zwischen zwei oder mehr Kanälen realisiert werden. Das Eindringen von Wasser kann keine Kurzschlüsse verursachen und muss somit nicht verhindert werden. Schließlich ist der erfindungsgemäße Verbinder wiederverwendbar, wodurch der mit der implantierten Elektrode verbundene Teil an Ort und Stelle verbleiben kann, wenn eine Reparatur der mit dem anderen Teil verbundenen Komponenten erfolgen muss.

**Bezugszeichenliste:**

| **Bezugsziffer** | **Beschreibung** |
|---|---|
| 100, 200, 300, 400 | Verbinderanordnung |
| 102, 202, 302, 402 | Erste Verbinder |
| 104, 204, 304, 404 | Zweite Verbinder |
| 106, 406 | Erstes Substrat |
| 108, 208, 408 | Erste Kopplungselektrode |
| 110, 410 | Zweites Substrat |
| 112, 212, 412, | Zweite Kopplungselektrode |
| 114 | Glättungskondensator |
| 116 | Erster Anschluss |
| 118 | Erste Passivierungsschicht |
| 120 | Zweite Passivierungsschicht |
| 122 | Zweiter Anschluss |
| 124 | Eingangssignal |
| 126 | Ausgabesignal |
| 128 | Justiervorsprung |
| 130 | Justierausnehmung |
| 132 | Trennspalt |
| 134 | Erstes lokales Dielektrikum |
| 136 | Zweites lokales Dielektrikum |
| 138, 238, 438 | Elektrische Leitung |
| 140 | Dritter Verbinder |
| 142 | Rückseitige Kopplungselektroden |
| 144 | Dritte Kopplungselektroden |
| 146 | Erste Substratseite |
| 148 | Zweite Substratseite |
| 150 | Vierte Kopplungselektroden |
| 152 | Drittes Substrat |
| 354 | Stiftförmiger Vorsprung |
| 356 | Buchsenförmige Ausnehmung |
| 458 | Elektronische Komponente |
| 460 | Via |
| 462 | Bondpad |
| 464 | Bonddraht |
| 466 | Gehäuse |

## Patentansprüche

1. Implantierbare elektrische Verbinderanordnung zum elektrischen Verbinden mindestens einer ersten und einer zweiten elektrischen Komponente, wobei die Verbinderanordnung (100) aufweist:
einen ersten Verbinder (102) mit mindestens einem ersten Anschluss (116) und mindestens einer ersten Kopplungselektrode (108), die mit dem ersten Anschluss (116) verbunden ist,
einen zweiten Verbinder (104) mit mindestens einem zweiten Anschluss (122) und mindestens einer zweiten Kopplungselektrode (112), die mit dem zweiten Anschluss (122) verbunden ist,
wobei der erste Verbinder (102) und der zweite Verbinder (104) so miteinander verbindbar sind, dass der erste und der zweite Anschluss (116, 122) über die erste Kopplungselektrode (108) und die zweite Kopplungselektrode (112) kapazitiv verbindbar sind,
**dadurch gekennzeichnet, dass**
im verbundenen Zustand zwischen der ersten Kopplungselektrode (108) und der zweiten Kopplungselektrode (112) ein definierter Trennspalt (132) gebildet ist, wobei der Trennspalt (132) nicht hermetisch gegenüber der Außenumgebung abgeschlossen ist.

2. Implantierbare elektrische Verbinderanordnung nach Anspruch 1, wobei der Trennspalt (132) wenigstens teilweise von einem Silikonmaterial begrenzt ist.

3. Implantierbare elektrische Verbinderanordnung nach einem der vorangehenden Ansprüche, wobei die Kopplungselektroden (108, 112) lokal mit einem elektrisch isolierenden Material (134, 136) beschichtet sind.

4. Implantierbare elektrische Verbinderanordnung nach einem der vorangehenden Ansprüche, weiterhin umfassend eine erste Justierstruktur (130), die an dem ersten Verbinder (102) angeordnet ist, und eine zweite Justierstruktur (128), die an dem zweiten Verbinder (104) angeordnet ist, wobei die erste und die zweite Justierstruktur zum Einstellen einer Breite des Trennspalts (132) im verbundenen Zustand zusammenwirken.

5. Implantierbare elektrische Verbinderanordnung nach einem der vorangehenden Ansprüche, wobei der erste Verbinder und/oder der zweite Verbinder einen integrierten Kondensator (114) aufweist.

6. Implantierbare elektrische Verbinderanordnung nach einem der vorangehenden Ansprüche, wobei der erste Verbinder und/oder der zweite Verbinder ein elektrisch isolierendes Substrat (106, 110) aufweist.

7. Implantierbare elektrische Verbinderanordnung nach einem der vorangehenden Ansprüche, wobei der erste Verbinder ein Array von ersten Kopplungselektroden aufweist, die im verbundenen Zustand mit einem Array von zweiten Kopplungselektroden des zweiten Verbinders verbunden sind.

8. Implantierbare elektrische Verbinderanordnung nach einem der vorangehenden Ansprüche, weiterhin aufweisend einen dritten Verbinder (140), mit mindestens einem dritten Anschluss und mindestens einer dritten Kopplungselektrode (144), die mit dem dritten Anschluss verbunden ist, wobei der zweite Verbinder mindestens einen vierten Anschluss und mindestens eine vierte Kopplungselektrode (142), die mit dem vierten Anschluss verbunden ist, aufweist, und wobei die dritte und vierte Kopplungselektrode (144, 142) im verbundenen Zustand der Verbinderanordnung kapazitiv gekoppelt sind und zwischen der dritten und vierten Kopplungselektrode ein zweiter Trennspalt (132) ausgebildet ist.

9. Implantierbare elektrische Verbinderanordnung nach Anspruch 8, wobei jeder der Verbinder (102, 104, 140) ein Substrat mit darauf angeordneten Kopplungselektroden aufweist, und wobei der zweite Verbinder (104) auf einer ersten Seite des Substrats (110) die zweiten Kopplungselektroden trägt und auf einer der ersten Seite gegenüberliegenden zweiten Seite die vierten Kopplungselektroden trägt.

10. Implantierbare elektrische Verbinderanordnung nach einem der vorangehenden Ansprüche, wobei der erste und der zweite Verbinder (202, 204) eingerollt sind, um jeweils eine zylindrische Raumform zu haben.

11. Implantierbare elektrische Verbinderanordnung nach einem der Ansprüche 1 bis 7, wobei der erste Verbinder (302) mindestens einen stiftförmigen Vorsprung (354) aufweist, an dem die mindestens eine erste Kopplungselektrode (308) angeordnet ist, und wobei der zweite Verbinder (304) mindestens eine Ausnehmung (356) zur wenigstens teilweisen Aufnahme des stiftförmigen Vorsprungs aufweist, wobei in der Ausnahme die mindestens eine zweite Kopplungselektrode (312) angeordnet ist.

12. Implantierbare elektrische Verbinderanordnung nach einem der vorangehenden Ansprüche, wobei mindestens einer der Verbinder weiterhin eine integrierte elektronische Komponente (458) aufweist.

13. Implantierbare Elektrodenanordnung, umfassend:
mindestens eine implantierbare Elektrode,
eine implantierbare Verbinderanordnung (100; 200; 300; 400) nach einem der vorangehenden Ansprüche,
wobei der mindestens eine erste Anschluss jeweils mit der mindestens einen Elektrode verbunden ist und der mindestens eine zweite Anschluss mit einer Ansteuerschaltung verbindbar ist.

14. Implantierbare Elektrodenanordnung nach Anspruch 13, wobei zwischen der mindestens einen implantierbaren Elektrode und der ersten Kopplungselektrode ein Glättungskondensator angeordnet ist (114).

## Claims

1. Implantable electrical connector arrangement for electrically connecting at least one first and a one second electrical component, said connector arrangement (100) comprising:
a first connector (102) with at least one first terminal (116) and at least one first coupling electrode (108) which is connected to said first terminal (116),
a second connector (104) with at least one second terminal (122) and at least one second coupling electrode (112) which is connected to said second terminal (122),
wherein said first connector (102) and said second connector (104) are connectable to each other such that said first and said second terminal (116, 122) are capacitively connectable via said first coupling electrode (108) and said second coupling electrode (112),
**characterized in that**
in the connected state, a defined separation gap (132) is formed between said first coupling electrode (108) and said second coupling electrode (112), wherein said separating gap (132) is not hermetically sealed against the outside environment.

2. Implantable electrical connector arrangement according to claim 1, wherein said separating gap (132) is defined at least in part by silicone material.

3. Implantable electrical connector arrangement according to one of the preceding claims, wherein said coupling electrodes (108, 112) are coated locally with electrically insulating material (134, 136).

4. Implantable electrical connector arrangement according to one of the preceding claims, further comprising a first adjustment structure (130) which is arranged on said first connector (102), and a second adjustment structure (128) which is arranged on said second connector (104), wherein said first and said second adjustment structure in the connected state interact for the adjustment of a width of said separating gap (132).

5. Implantable electrical connector arrangement according to one of the preceding claims, wherein said first connector and/or said second connector comprises an integrated capacitor (114).

6. Implantable electrical connector arrangement according to one of the preceding claims, wherein said first connector and/or said second connector comprise an electrically insulating substrate (116, 110).

7. Implantable electrical connector arrangement according one of the preceding claims, wherein said first connector comprises an array of first coupling electrodes which in the connected state are connected to an array of second coupling electrodes of said second connector.

8. Implantable electrical connector arrangement according to one of the preceding claims, further comprising a third connector (140) with at least one third terminal and at least one third coupling electrode (144) which is connected to said third terminal, wherein said second connector comprises at least one fourth terminal and a fourth coupling electrode (142) which is connected to said fourth terminal, and wherein said third and fourth coupling electrodes (144, 142) in the connected state of said connector arrangement are capacitively coupled and a second separating gap (132) is formed between said third and fourth coupling electrodes.

9. Implantable electrical connector arrangement according to claim 8, wherein each of said connectors (102, 104, 140) comprises a substrate with coupling electrodes arranged thereon, and wherein said second connector (104) carries said second coupling electrodes on a first side of said substrate (110) and carries said fourth coupling electrodes on a second side disposed opposite said first side.

10. Implantable electrical connector arrangement according to one of the preceding claims, wherein said first and said second connector (202, 204) are curled up in order to each have a cylindrical three-dimensional shape.

11. Implantable electrical connector arrangement according to one of the claims 1 to 7, wherein said first connector (302) comprises at least one pin-shaped protrusion (354) on which said at least one first coupling electrode (308) is arranged, and
wherein said second connector (304) comprises at least one recess (356) for receiving said pin-shaped protrusion at least in part, wherein said at least one second coupling electrode (312) is arranged in said recess.

12. Implantable electrical connector arrangement according to one of the preceding claims, wherein at least one of said connectors further comprises an integrated electronic component (458).

13. Implantable electrical connector arrangement comprising: at least one implantable electrode,
an implantable connector arrangement (100; 200; 300; 400) according to one of the preceding claims,
wherein said at least one first terminal is connected to said at least one electrode and said at least one second terminal is connectable to an actuation circuit.

14. Implantable electrode arrangement according to claim 13, wherein a smoothing capacitor is arranged (114) between said at least one implantable electrode and said first coupling electrode.

## Revendications

1. Ensemble de connecteurs électriques implantables pour connecter électriquement au moins un premier et un deuxième composant électrique, l'ensemble de connecteurs (100) comprenant :
un premier connecteur (102) ayant au moins une première borne (116) et au moins une première électrode de couplage (108) connectée à la première borne (116),
un deuxième connecteur (104) ayant au moins une deuxième borne (122) et au moins une deuxième électrode de couplage (112) connectée à la deuxième borne (122),
dans lequel le premier connecteur (102) et le second connecteur (104) peuvent être connectés l'un à l'autre de telle sorte que les première et seconde bornes (116, 122) peuvent être connectées de manière capacitive via la première électrode de couplage (108) et la seconde électrode de couplage (112),
**caractérisé en ce que**, à l'état connecté, un espace de séparation défini (132) est formé entre la première électrode de couplage (108) et la deuxième électrode de couplage (112), l'espace de séparation (132) n'étant pas fermé hermétiquement vis-à-vis de l'environnement extérieur.

2. Ensemble de connecteurs électriques implantables selon la revendication 1, dans lequel l'espace de séparation (132) est au moins partiellement délimité par un matériau en silicone.

3. Ensemble de connecteurs électriques implantables selon l'une quelconque des revendications précédentes, dans lequel les électrodes de couplage (108, 112) sont revêtues localement d'un matériau électriquement isolant (134, 136).

4. Ensemble de connecteurs électriques implantables selon l'une quelconque des revendications précédentes, comprenant en outre une première structure d'ajustement (130) disposée sur le premier connecteur (102) et une seconde structure d'ajustement (128) disposée sur le second connecteur (104), la première et la seconde structure d'ajustement coopérant pour ajuster une largeur de l'espace de séparation (132) à l'état connecté.

5. Ensemble de connecteurs électriques implantables selon l'une quelconque des revendications précédentes, dans lequel le premier connecteur et/ou le deuxième connecteur comprennent un condensateur intégré (114).

6. Ensemble de connecteurs électriques implantables selon l'une quelconque des revendications précédentes, dans lequel le premier connecteur et/ou le deuxième connecteur comprennent un substrat électriquement isolant (106, 110).

7. Ensemble de connecteurs électriques implantables selon l'une quelconque des revendications précédentes, dans lequel le premier connecteur comprend un réseau de premières électrodes de couplage qui, à l'état connecté, sont connectées à un réseau de secondes électrodes de couplage du second connecteur.

8. Ensemble de connecteurs électriques implantables selon l'une quelconque des revendications précédentes, comprenant en outre un troisième connecteur (140), comportant au moins une troisième borne et au moins une troisième électrode de couplage (144) connectée à la troisième borne, dans lequel le deuxième connecteur comporte au moins une quatrième borne et au moins une quatrième électrode de couplage (142), qui est connectée à la quatrième borne, et dans lequel la troisième et la quatrième électrode de couplage (144, 142) sont couplées de manière capacitive dans l'état connecté de l'ensemble de connecteurs, et un deuxième espace de séparation (132) est formé entre la troisième et la quatrième électrode de couplage.

9. Ensemble de connecteurs électriques implantables selon la revendication 8, dans lequel chacun desdits connecteurs (102, 104, 140) comprend un substrat sur lequel sont disposées des électrodes de couplage, et dans lequel ledit second connecteur (104) porte lesdites secondes électrodes de couplage sur un premier côté dudit substrat (110) et porte lesdites quatrièmes électrodes de couplage sur un second côté opposé audit premier côté.

10. Ensemble de connecteurs électriques implantables selon l'une quelconque des revendications précédentes, dans lequel le premier et le deuxième connecteur (202, 204) sont enroulés afin de présenter à chaque fois une forme spatiale cylindrique.

11. Ensemble de connecteurs électriques implantables selon l'une quelconque des revendications 1 à 7, dans lequel le premier connecteur (302) comprend au moins une protubérance en forme de broche (354) sur laquelle est disposée la au moins une première électrode de couplage (308), et dans lequel le deuxième connecteur (304) comprend au moins un évidement (356) pour recevoir au moins partiellement la protubérance en forme de broche, la au moins une deuxième électrode de couplage (312) étant disposée dans l'évidement.

12. Ensemble de connecteurs électriques implantables selon l'une quelconque des revendications précédentes, dans lequel au moins l'un desdits connecteurs comprend en outre un composant électronique intégré (458).

13. Ensemble d'électrodes implantables, comprenant :
au moins une électrode implantable,
un ensemble de connecteurs implantables (100 ; 200 ; 300 ; 400) selon l'une quelconque des revendications précédentes,
l'au moins une première connexion étant respectivement reliée à l'au moins une électrode et l'au moins une deuxième connexion pouvant être reliée à un circuit de commande.

14. Ensemble d'électrodes implantables selon la revendication 13, dans lequel un condensateur de lissage est disposé (114) entre ladite au moins une électrode implantables et ladite première électrode de couplage.
